# EUROPEAN PATENT APPLICATION

(11) **EP 1 607 100 A1**
(43) Date of publication of application: **21.12.2005**
(21) Application number: 04714014.0
(22) Date of filing: 24.02.2004
(51) Int. Cl.: A61K 45/00, A61K 39/395, A61P 19/00, A61P 25/00, A61P 43/00

(54) **REMEDY FOR SPINAL INJURY CONTAINING INTERLEUKIN-6 ANTAGONIST**

(30) Priority: 24.02.2003 JP 2003046214
(71) Applicant: CHUGAI SEIYAKU KABUSHIKI KAISHA, Tokyo, 115-8543 (JP); Keio University, Tokyo 108-8345 (JP)
(72) Inventor: OKANO, Hideyuki Keio University School Of Medicine, Tokyo 1608582 (JP); OKADA, Seiji, Keio University School Of Medicine, Tokyo 1608582 (JP); NAKAMURA, M., Keio University School Of Medicine, Tokyo 1608582 (JP); YOSHIZAKI, Kazuyuki, Kobe-shi, Hyogo 6580016 (JP)
(74) Representative: Harding, Charles Thomas
(86) International application number: PCT/JP2004/002111
(87) International publication number: WO 2004/073741

(57) **Abstract**

A therapeutic agent for spinal cord injury, a modulator of differentiation of neural stem cells and an inhibitor of differentiation into glia cells comprising an interleukin-6 antagonist as an active ingredient.

## Description

### Technical Field

The present invention relates to a therapeutic agent for spinal cord injury comprising interleukin-6 (IL-6) antagonist as an active ingredient.

### Background Art

In today's society, a person may suffer spinal cord injury due to a motor vehicle accident, a fall, a tumble, a sports injury and the like. In Japan, the annual number of the injured people is about 5,000 with a cumulative number of patients possibly amounting to 100,000. Symptoms of spinal cord injury are very severe including permanent quadriplegia, motor paralysis and sensory paralysis, bladder and rectum disorders, respiratory disorders etc., and the daily management thereof includes rehabilitation, respiratory management, bedsore prevention, the management of defecation and urination, and the like.

As therapeutic regimens for spinal cord injury, no effective methods of treatment are currently available and there are only symptomatic treatments including local stabilization such as surgery. In order to prevent the aggravation of pathological conditions, steroids have been administered in large quantities, but no results have been obtained that indicate recovery from paralysis. Many spinal cord injuries start with an injury (primary injury) by an external mechanical action and progress further to tissue destruction (secondary injury) through reaction pathways in the living body. The only drug for use in inhibiting the progression of such secondary injuries is methyl prednisolone, which has been administered in quantities as large as nearly 10000 mg. It has been reported, however, that this method is associated with side effects such as the aggravation of diabetes mellitus and pneumonia.

In the beginning of the 19th century, Ramon y Cajal, a neuroanatomist, stated in his book "The central nervous system (the brain and the spinal cord) of mammals is incapable of regeneration once it has been injured," and since then this has been believed. In 1980's, however, the implantation of peripheral nerves for spinal cord injury (A. Aguayo et al., J. Exp. Bilo. 95:231-240, 1981) and the implantation of fetus spinal cord (Bregman B.S., Dev. Brain Res., 34:265-279, 1987) have been reported, indicating that even after spinal cord injury the regeneration of injured axons can be observed if an appropriate environment has been introduced at the injured site. Also, a multiplicity of reports on axonal regeneration have been made such as the promotion of regeneration of injured axons by neurotrophic factors (Cai, D. et al., Neuron, 22:89-101, 1999), the identification of inhibitory factors of axonal growth (Chen, D. et al., Nature 403:434-439, 2000) and the like, suggesting that the regeneration of injured spinal cord injury may become a reality. However, the clinical application of the implantation of the fetus spinal cord is very difficult due to the shortage of donors and to ethical problems.

Furthermore, neural stem cells are (pluripotent) undifferentiated cells of the nervous system that can propagate and repeated passages (self-replicating ability) and simultaneously generate three types of cells (neurons, astrocytes, oligodendrocytes) that constitute the central nervous system and, as they also occur in the adult spinal cord, they are assumed to be capable of repairing injured tissues. In fact, however, they do not differentiate into neurons after injury, and all differentiate into glia cells thereby forming scars.

There are sporadic reports on cytokines involved in differentiation induction in neural stem cells. Weiss et al. reported that the differentiation of neural stem cells derived from the striate body of a mouse fetus into neurons is promoted by brain-derived neurotrophic factor (BDNF) (Ahmed, S. et al., J. Neurosci. 150:5765-5778, 1995). Ghosh et al. also reported that the differentiation of neural stem cells derived from the cerebral skin of a rat fetus into neurons is promoted by neurotrophin-3 (NT-3) (Ghosh, A. et al., Neuron 15:89-103, 1995). McKay et al. reported that the differentiation of neural stem cells derived from the hippocampus of a rat fetus is "instructively" induced into neurons by platelet-derived neurotrophic factor (PDNF), into astrocytes by ciliary neurotrophic factor (CNTF), and into oligodendrocytes by thyroid hormone (T3) (Jone, K. et al., Gene & Dev. 10:3129-3140).

Furthermore, Taga et al. recently reported that the differentiation of neural stem cells derived from the neural epithelial cells of a mouse fetus into astrocytes is promoted by leukemia inhibitory factor (LIF) and bone morphogenic protein-2 (BMP-2) (Nakashima et al., Science 284:479-482, 1999). Common to these reports are the so-called IL-6 superfamily such as CNTF and LIF. Thus, a signal via gp130, which is a subunit of the cytokine receptor, is believed to induce the differentiation of neural stem cells into astrocytes.

However, there is no literature that demonstrates that spinal cord injury can be repaired by the differentiation induction of neural stem cells by cytokines.

IL-6 is a cytokine which is also called B cell stimulating factor 2 (BSF2) or interferon β2. IL-6 was discovered as a differentiation factor involved in the activation of B-lymphatic cells (Hirano, T. et al., Nature (1986) 324, 73-76). Thereafter, it was found to be a multifunctional cytokine that influences various functions of the cell (Akira, S. et al., Adv. in Immunology (1993) 54, 1-78). IL-6 has been reported to induce the maturation of T-lymphatic cells (Lotz, M. et al., J. Exp. Med. (1988) 167, 1253-1258).

IL-6 transmits its biological activity through two types of proteins on the cell. One of them is IL-6 receptor, a ligand-biding protein with a molecular weight of about 80 kD, to which IL-6 binds (Taga, T. et al., J. Exp. Med. (1987) 166, 967-981; Yamasaki, K. et al., Science (1987) 241, 825-828). IL-6 receptor occurs not only in the membrane-bound form that penetrates through and is expressed on the cell membrane but also as a soluble IL-6 receptor consisting mainly of the extracellular region.

The other is a membrane-bound protein gp130 having a molecular weight of about 130 kD that is involved in non-ligand-binding signal transduction. IL-6 and IL-6 receptor form the IL-6/IL-6 receptor complex which, after binding to gp130, transmits the biological activity of IL-6 to the cell (Taga, T. et al., Cell (1989) 58, 573-581) .

An IL-6 antagonist is a substance that inhibits the transduction of biological activity of IL-6. There have been known so far antibody directed against IL-6 (anti-IL-6 antibody), antibody directed against IL-6 receptor (anti-IL-6 receptor antibody), antibody directed against gp130 (anti-gp130 antibody), altered IL-6, partial peptides of IL-6 or IL-6 receptor and the like.

Anti-IL-6 receptor antibody has been described in several reports (Novick D. et al., Hybridoma (1991) 10, 137-146, Huang, Y. W. et al., Hybridoma (1993) 12, 621-630, International Patent Publication WO 95-09873, French Patent Application FR 2694767, United States Patent US 521628). Humanized PM-1 antibody was obtained by transplanting the complementarity determining region (CDR) of one of them, a mouse antibody PM-1 (Hirata, Y. et al., J. Immunology (1989) 143, 2900-2906), to a human antibody (the International Patent Publication WO 92-19759).
Patent document 1: WO95-09873
Patent document 2: FR 2694767
Patent document 3: USP 0521628
Non-patent document 1: A. Aguayo et al., J. Exp. Bilo. 95:231-240, 1981
Non-patent document 2: Bregman B.S., Dev. Brain Res. 34:265-279, 1987
Non-patent document 3: Cai, D. et al., Neuron 22:89-101, 1999
Non-patent document 4: Chen, D. et al., Nature 403:434-439, 2000
Non-patent document 5: Ahmed, S. et al., J. Neurosci. 150:5765-5778, 1995
Non-patent document 6: Ghosh, A. et al., Neuron 15:89-103, 1995
Non-patent document 7: Jone, K. et al., Gene & Dev. 10:3129-3140, 1996
Non-patent document 8: Nakashima, K. et al., Science 284:479-482, 1999
Non-patent document 9: Hirano, T. et al., Nature (1986) 324, 73-76
Non-patent document 10: Akira, S. et al., Adv. in Immunology (1993) 54, 1-78
Non-patent document 11: Lotz, M. et al., J. Exp. Med. (1988) 167, 1253-1258
Non-patent document 12: Taga, T. et al., Cell (1989) 58, 573-581
Non-patent document 13: Yamasaki, K. et al., Science (1987) 241, 825-828
Non-patent document 14: Novick, D. et al., Hybridoma (1991) 10, 137-146
Non-patent document 15: Huang, Y.W. et al., Hybridoma (1993) 12, 621-630
Non-patent document 16: Hirata, Y. et al., J. Immunol. (1989) 143, 2900-2906

### Disclosure of the Invention

Thus, there is a need for a therapeutic means that not only prevents the aggravation of conditions of a spinal cord injury but also aids recovery therefrom, and the present invention provides a pharmaceutical composition useful as one of the means.

After intensive and extensive studies to resolve the above problem, the present inventors have found that agonist against IL-6, for example, antibody against IL-6 receptor has an effect of aiding recovery from spinal cord injury. Thus, the present invention provides a therapeutic agent for spinal cord injury comprising interleukin-6 (IL-6) antagonist as an active ingredient.

The present invention also provides a modulator of differentiation of neural stem cells comprising interleukin-6 (IL-6) antagonist as an active ingredient.

The present invention also provides an inhibitor of differentiation into glia cells comprising interleukin-6 (IL-6) antagonist as an active ingredient.

The above IL-6 antagonist is preferably an antibody against IL-6 receptor, and most preferably a monoclonal antibody. As such a monoclonal antibody, there can be mentioned, for example, a monoclonal antibody against human IL-6 receptor and a monoclonal antibody against mouse IL-6 receptor. As a specific example of the above monoclonal antibody against human IL-6 receptor, there can be mentioned for example PM-1 antibody, and as a specific example of the above monoclonal antibody against mouse IL-6 receptor, there can be mentioned for example MR16-1 antibody. Furthermore, as an antibody against IL-6 receptor, there can be mentioned a recombinant antibody, for example, a chimeric antibody, a humanized antibody, and the like, that has been obtained by artificially engineering a gene cloned from a monoclonal antibody-producing hybridoma.

### Brief Explanation of the Drawings

Fig. 1 is a graph showing that the recovery of motion after spinal cord injury is greater in the spinal cord injured mice that received an anti-IL-6 receptor antibody (MR16) as compared to the spinal cord injured mice (control) that did not receive the above antibody in the evaluation of motor function of the lower limbs.

Fig. 2 is a graph showing that the recovery of motion coordination after spinal cord injury is greater in the spinal cord injured mice that received an anti-IL-6 receptor antibody (MR16) as compared to the spinal cord injured mice (control) that did not receive the above antibody in the rotarod treadmill test.

Fig. 3 is a graph showing that glia formation at the spinal cord injured site has been inhibited in the spinal cord injured mice that received an anti-IL-6 receptor antibody (MR16) as compared to the spinal cord injured mice (control) that did not receive the above antibody.

Fig. 4 is a graph showing that IL-6 receptor has been expressed at the spinal cord injured site in the spinal cord injured mice as compared to the mice (sham) that have no spinal cord injury.

Fig. 5 is the Western blot of a phosphorylated STAT3 showing that the administration of an anti-IL-6 receptor antibody (MR16) actually inhibited the IL-6 signal cascade at the spinal cord injured site.

### Best Mode for Carrying out the Invention

IL-6 antagonists for use in the present invention may be of any origin, any kind, and any form, as long as they exhibit a therapeutic effect on spinal cord injury.

IL-6 antagonists block signal transduction by IL-6 and inhibit the biological activity of IL-6. IL-6 antagonists are preferably substances that have an activity of inhibiting the binding to any of IL-6, IL-6 receptor, and gp130. As the IL-6 antagonists, there can be mentioned for example anti-IL-6 antibody, anti-IL-6 receptor antibody, anti-gp130 antibody, altered IL-6, altered soluble IL-6 receptor, a partial peptide of IL-6 or IL-6 receptor, and a low molecular weight substance having the same activity as these.

Anti-IL-6 antibodies for use in the present invention can be obtained as polyclonal or monoclonal antibodies using a known method. As the anti-IL-6 antibodies for use in the present invention, monoclonal antibodies of, in particular, a mammalian origin, are preferred. Monoclonal antibodies of a mammalian origin include those produced by a hybridoma and recombinant antibody produced by a host which has been transformed with an expression vector containing genetically engineered antibody genes. These antibodies, via binding to IL-6, block the binding of IL-6 to IL-6 receptor, and thereby block the signal transduction of biological activity of IL-6 into the cell.

Examples of such antibodies include MH166 (Matsuda T. et al., Eur. J. Immunol. (1988) 18, 951-956) and SK2 antibody (Sato, K. et al., The 21st Nihon Menekigakkai Soukai (General Meeting of the Japan Immunology Society), Academic Record (1991) 21, 166) and the like.

An anti-IL-6 antibody-producing hybridoma can be basically constructed using a known procedure as described below. Thus, IL-6 may be used as a sensitizing antigen and is immunized in the conventional method of immunization. The immune cells thus obtained are fused with known parent cells in the conventional cell fusion process, and then monoclonal antibody-producing cells are screened by the conventional screening method to prepare the desired hybridoma.

Specifically, anti-IL-6 antibody may be obtained in the following manner. For example, a human IL-6 for use as the sensitizing antigen to obtain antibody can be obtained using the IL-6 gene/amino acid sequence disclosed in Eur. J. Biochem (1987) 168, 543-550, J. Immunol. (1988) 140, 1534-1541, or Agr. Biol. (1990) 54, 2685-2688.

After a suitable host cell is transformed by inserting the IL-6 gene sequence into a known expression vector system, the IL-6 protein of interest is purified from the host cell or the culture supernatant thereof, and the purified IL-6 protein can be used as the sensitizing antigen. Alternatively, a fusion protein, of the IL-6 protein and another protein, may be used as the sensitizing antigen.

Anti-IL-6 receptor antibodies for use in the present invention can be obtained as polyclonal or monoclonal antibodies using a known method. As the anti-IL-6 receptor antibodies for use in the present invention, monoclonal antibodies of, in particular a mammalian origin, are preferred. Monoclonal antibodies of a mammalian origin include those produced by a hybridoma and those produced by a host which has been transformed with an expression vector containing genetically engineered antibody genes. The antibodies, via binding to IL-6 receptor, inhibit the binding of IL-6 to IL-6 receptor, and thereby block the transduction of the biological activity of IL-6 into the cell.

Examples of such antibodies include MR16-1 antibody (Tamura, T., et al., Proc. Natl. Acad. Sci. USA (1993) 90, 11924-11928), PM-1 antibody (Hirata, Y. et al., J. Immunol. (1989) 143, 2900-2906), or AUK12-20 antibody, AUK64-7 antibody or AUK146-15 antibody (International Patent Publication WO 92-19759) and the like. Among them, PM-1 antibody is most preferred.

Incidentally, the hybridoma cell line which produces PM-1 antibody has been internationally deposited under the provisions of the Budapest Treaty as PM-1 on July 12, 1989 with the Patent Microorganism Depository of National Institute of Industrial Science and Technology, of Chuo 6, 1-1, Higashi 1-chome, Tsukuba city, Ibaraki pref., Japan, as FERM BP-2998. The hybridoma cell line which produces MR16-1 antibody has been internationally deposited under the provisions of the Budapest Treaty as Rat-mouse hybridoma MR16-1 on March 13, 1997 with the Patent Microorganism Depository of National Institute of Industrial Science and Technology, of Chuo 6, 1-1, Higashi 1-chome, Tsukuba city, Ibaraki pref., Japan, as FERM BP-5875.

Hybridomas producing anti-IL-6 receptor monoclonal antibody can be basically prepared using a known procedure as described below. Thus, IL-6 receptor is used as a sensitizing antigen and is immunized according to the conventional method of immunization. The immune cells thus obtained are fused with known parent cells in the conventional cell fusion process, and then monoclonal antibody-producing cells may be screened by the conventional screening method to prepare the desired hybridoma .

Specifically, anti-IL-6 receptor antibody may be prepared in the following manner. For example, the human IL-6 receptor used as the sensitizing antigen for obtaining antibody can be obtained using the IL-6 receptor gene sequence/amino acid sequence disclosed in European Patent Application EP 325474, and the mouse IL-6 receptor can be obtained using the IL-6 receptor gene sequence, amino acid sequence disclosed in Japanese Unexamined Patent Publication (Kokai) 3-155795.

There are two types of IL-6 receptor proteins: IL-6 receptor expressed on the cell membrane, and IL-6 receptor detached from the cell membrane (soluble IL-6 receptor) (Yasukawa K. et al., J. Biochem. (1990) 108, 673-676). Soluble IL-6 receptor antibody is composed substantially of the extracellular region of the IL-6 receptor bound to the cell membrane, and thereby is different from the membrane-bound IL-6 receptor in that the former lacks the transmembrane region or both of the transmembrane region and the intracellular region. As the IL-6 receptor protein, any IL-6 receptor can be used, as long as it can be used a sensitizing antigen for production of the anti-IL-6 receptor antibody for use in the present invention.

After the gene sequence of IL-6 receptor is inserted into a known expression vector system to transform an appropriate host cell, the desired IL-6 receptor protein may be purified from the host cell or a culture supernatant thereof using a known method, and the purified IL-6 receptor protein may be used as the sensitizing antigen. Alternatively, cells that are expressing IL-6 receptor or a fusion protein of the IL-6 receptor protein and another protein may be used as the sensitizing antigen.

Escherichia coli (E. coli) that has a plasmid pIBIBSF2R containing cDNA encoding human IL-6 receptor has been internationally deposited under the provisions of the Budapest Treaty as HB101-pIBIBSF2R on January 9, 1989 with the Patent Microorganism Depository of National Institute of Industrial Science and Technology, of Chuo 6, 1-1, Higashi 1-chome, Tsukuba city, Ibaraki pref., Japan, as FERM BP-2232.

Anti-gp130 antibodies for use in the present invention can be obtained as polyclonal or monoclonal antibodies using a known method. As the anti-gp130 antibodies for use in the present invention, monoclonal antibodies of, in particular a mammalian origin, are preferred. Monoclonal antibodies of a mammalian origin include those produced by a hybridoma and those produced by a host which has been transformed with an expression vector containing genetically engineered antibody genes. The antibodies, via binding to gp130, inhibit the binding of IL-6/IL-6 receptor complex to gp130, and thereby block the transduction of the biological activity of IL-6 into the cell.

Examples of such antibodies include AM64 antibody (Japanese Unexamined Patent Publication (Kokai) 3-219894), 4B11 antibody and 2H4 antibody (US 5571513), B-S12 antibody and B-P8 antibody (Japanese Unexamined Patent Publication (Kokai) 8-291199) and the like.

An anti-gp130 monoclonal antibody-producing hybridoma can be basically created using a known procedure as described below. Thus, gp130 may be used as a sensitizing antigen and is used for immunizing a conventional method of immunization. The immune cells thus obtained are fused with known parent cells in a conventional cell fusion process, and then the monoclonal antibody-producing hybridomas are screened by a conventional screening method to prepare the desired hybridoma.

Specifically, monoclonal antibody may be obtained in the following manner. For example, gp130 used as the sensitizing antigen for antibody generation can be obtained using the gp130 gene sequence/amino acid sequence disclosed in European Patent Application EP 411946.

After a suitable host cell is transformed by inserting the gp130 gene sequence into a known expression vector system, the gp130 protein of interest is purified from the host cell or from the culture supernatant thereof in a conventional method. The purified gp130 receptor protein can be used as the sensitizing antigen. Alternatively, cells expressing gp130 or a fusion protein, of the gp130 protein and another protein, may be used as the sensitizing antigen.

Though the mammals to be immunized with the sensitizing antigen are not specifically limited, they are preferably selected in consideration of their compatibility with the parent cell for use in cell fusion. They generally include rodents such as mice, rats, hamsters and the like.

Immunization of animals with a sensitizing antigen is carried out using a known method. A general method, for example, involves the intraperitoneal or subcutaneous injection of a sensitizing antigen to the mammal. Specifically, a sensitizing antigen which has been diluted and suspended in an appropriate amount of phosphate buffered saline (PBS) or physiological saline etc. is mixed, as desired, with an appropriate amount of a common adjuvant, for example Freund's complete adjuvant. After being emulsified, it is preferably administered to a mammal for several times every 4 to 21 days. Alternatively a suitable carrier may be used at the time of immunization of the sensitizing antigen.

After immunization and the confirmation of the increase in the desired antibody levels in the serum, the immune cells are taken out from the mammal and are subjected to cell fusion. Preferred immune cells to be subjected to cell fusion include, in particular, the spleen cells.

The mammalian myeloma cells, as the other parent cells which are fused with the above-mentioned immune cells, preferably include various known cell lines such as P3X63Ag8.653 (Kearney, J. F. et al., J. Immunol. (1979) 123, 1548-1550), P3X63Ag8U.1 (Current Topics in Microbiology and Immunology (1978) 81, 1-7), NS-1 (Kohler, G. and Milstein, C., Eur. J. Immunol. (1976) 6, 511-519), MPC-11 (Margulies, D.H. et al., Cell (1976) 8, 405-415), SP2/0 (Shulman, M. et al., Nature (1978) 276, 269-270), FO (de St. Groth, S. F. et al., J. Immunol. Methods (1980) 35; 1-21), S194 (Trowbridge, I.S., J. Exp. Med. (1978) 148, 313-323), R210 (Galfre, G. et al., Nature (1979) 277; 131-133) and the like.

Cell fusion between the above immune cells and the myeloma cells may be essentially conducted in accordance with a known method such as is described in Milstein et al. (Kohler, G. and Milstein, C., Methods Enzymol. (1981) 73, 3-46) and the like.

More specifically, the above cell fusion is carried out in the conventional nutrient broth in the presence of, for example, a cell fusion accelerator. As the cell fusion accelerator, for example, polyethylene glycol (PEG), Sendai virus (HVJ) and the like may be used, and, in addition, an adjuvant such as dimethyl sulfoxide etc. may be added as desired to enhance the efficiency of fusion.

The preferred ratio of the immune cells and the myeloma cells to be used is, for example, 1 to 10 times more immune cells than the myeloma cells. Examples of culture media to be used for the above cell fusion include RPMI1640 medium and MEM culture medium suitable for the growth of the above myeloma cell lines, and the conventional culture medium used for this type of cell culture, and besides a serum supplement such as fetal calf serum (FCS) may be added.

In cell fusion, predetermined amounts of the above immune cells and the myeloma cells are mixed well in the above culture liquid, to which a PEG solution previously heated to about 37°C, for example a PEG solution with a mean molecular weight of about 1000 to 6000, is added at a concentration of 30 to 60%(w/v) and mixed to form the desired fusion cells (hybridoma). Then by repeating the sequential addition of a suitable culture liquid and centrifugation to remove the supernatant, cell fusion agents etc. which are undesirable for the growth of the hybridoma can be removed.

Said hybridoma may be selected by culturing in the conventional selection medium, for example, the HAT culture medium (a culture liquid containing hypoxanthine, aminopterin, and thymidine). Culturing in said HAT culture liquid is continued generally for a period of time sufficient to effect killing of the cells (non-fusion cells) other than the desired hybridoma, and generally for several days to several weeks. Then the conventional limiting dilution method is conducted to effect the screening and cloning of the hybridomas that produce the desired antibody.

In addition to obtaining the above hybridoma by immunizing an animal other than the human with an antigen, it is also possible to sensitize human lymphocytes in vitro with a desired antigen protein or desired antigen-expressing cells, and the resulting sensitized B lymphocytes are fused with human myeloma cells, for example U266, to obtain the desired human antibody having the activity of binding to the desired antigen or the desired antigen-expressing cells (see Japanese Post-examined Patent Publication (Kokoku) No. 1-59878). Furthermore, a transgenic animal having a repertoire of all human antibody genes can be immunized with the antigen or the antigen-expressing cells to obtain the desired human antibody in the method described above (see International Patent Publication WO 93/12227, WO 92/03918, WO 94/02602, WO 94/25585, WO 96/34096 and WO 96/33735).

The monoclonal antibody-producing hybridoma thus constructed can be subcultured in the conventional culture liquid, or can be stored for a prolonged period of time in liquid nitrogen.

In order to obtain monoclonal antibodies from said hybridoma, a method can be used in which said hybridoma is cultured in the conventional method and the antibodies are obtained as the supernatant, or a method in which the hybridoma is administered to and grown in a mammal compatible with said hybridoma and the antibodies are obtained as the ascites. The former method is suitable for obtaining high-purity antibodies, whereas the latter is suitable for a large scale production of antibodies.

For example, a hybridoma producing anti-IL-6 receptor antibody can be constructed using the method disclosed in Japanese Unexamined Patent Publication (Kokai) 3-139293. It can be constructed by a method in which the PM-1 antibody-producing hybridoma that was internationally deposited under the provisions of the Budapest Treaty as FERM BP-2998 on July 12, 1989 with the Patent Microorganism Depository of National Institute of Industrial Science and Technology, of Chuo 6, 1-1, Higashi 1-chome, Tsukuba city, Ibaraki pref., Japan, is intraperitoneally injected to BALB/c mice to obtain the ascites from which the PM-1 antibody is purified, or a method in which said hybridoma is cultured in a suitable culture medium such as the RPMI1640 medium containing 10% bovine fetal serum and 5% MB-Condimed H1 (manufactured by Boehringer Mannheim), the hybridoma SFM medium (manufactured by GIBCO-BRL), the PFHM-II medium (manufactured by GIBCO-BRL) and the like, and the PM-1 antibody can be purified from the supernatant.

A recombinant antibody which was produced by the recombinant gene technology in which an antibody gene was cloned from the hybridoma and integrated into a suitable vector which was then introduced into a host can be used in the present invention as monoclonal antibody (see, for example, Borrebaeck C.A.K., and Larrick J.W. THERAPEUTIC MONOCLONAL ANTIBODIES, published in the United Kingdom by MACMILLAN PUBLISHERS LTD. 1990).

Specifically, mRNA encoding the variable (V) region of the desired antibody is isolated from antibody-producing cells such as a hybridoma. The isolation of mRNA is conducted by preparing total RNA using, for example, a known method such as the guanidine ultracentrifuge method (Chirgwin, J.M. et al., Biochemistry (1979) 18, 5294-5299), the AGPC method (Chomczynski, P. et al., Anal. Biochem. (1987) 162, 156-159), and then mRNA is prepared from the total RNA using the mRNA Purification kit (manufactured by Pharmacia) and the like. Alternatively, mRNA can be directly prepared using the QuickPrep mRNA Purification Kit (manufactured by Pharmacia).

cDNA of the V region of antibody may be synthesized from the mRNA thus obtained using a reverse transcriptase. cDNA may be synthesized using the AMV Reverse Transcriptase First-strand cDNA Synthesis Kit and the like. Alternatively, for the synthesis and amplification of cDNA, the 5'-Ampli FINDER RACE Kit (manufactured by Clontech) and the 5'-RACE method (Frohman, M.A. et al., Proc. Natl. Acad. Sci. USA (1988) 85, 8998-9002; Belyavsky, A. et al., Nucleic Acids Res. (1989) 17, 2919-2932) that employs polymerase chain reaction (PCR) may be used. The desired DNA fragment is purified from the PCR product obtained and may be ligated to vector DNA. Moreover, a recombinant vector is constructed therefrom and then is introduced into E. coli etc., from which colonies are selected to prepare the desired recombinant vector. The base sequence of the desired DNA may be confirmed by a known method such as the dideoxy method.

Once the DNA encoding the V region of the desired antibody has been obtained, it may be ligated to DNA encoding the constant region (C region) of the desired antibody, which is then integrated into an expression vector. Alternatively, the DNA encoding the V region of the antibody may be integrated into an expression vector containing DNA encoding the C region of the antibody.

In order to produce the antibody for use in the present invention, the antibody gene is integrated as described below into an expression vector so as to be expressed under the control of the expression regulatory region, for example an enhancer and/or a promoter. Subsequently, the expression vector may be transformed into a host cell and the antibody can then be expressed therein.

In accordance with the present invention, artificially altered recombinant antibody such as chimeric antibody, humanized antibody, and human antibody can be used for the purpose of lowering heterologous antigenicity against humans. These altered antibodies can be produced using known methods.

Chimeric antibody can be obtained by ligating the thus obtained DNA encoding the V region of antibody to DNA encoding the C region of human antibody, which is then integrated into an expression vector and introduced into a host for production of the antibody therein (see European Patent Application EP 125023, and International Patent Publication WO 92-19759). Using this known method, chimeric antibody useful for the present invention can be obtained.

For example, a plasmid that contains DNA encoding the L chain V region or the H chain V region of chimeric PM-1 antibody was designated as pPM-k3 or pPM-h1, respectively, and E. coli's having these plasmids have been internationally deposited under the provisions of the Budapest Treaty as NCIMB 40366 and NCIMB 40362, respectively, on February 12, 1991 with the National Collections of Industrial and Marine Bacteria Limited.

Humanized antibody which is also called reshaped human antibody has been made by transplanting the complementarity determining region (CDR) of antibody of a mammal other than the human, for example mouse antibody, into the complementarity determining region of human antibody. The general recombinant DNA technology for preparation of such antibodies is also known (see European Patent Application EP 125023 and International Patent Publication WO 92-19759).

Specifically, a DNA sequence which was designed to ligate the CDR of mouse antibody with the framework region (FR) of human antibody is synthesized from several divided oligonucleotides having sections overlapping with one another at the ends thereof by the PCR method. The DNA thus obtained is ligated to the DNA encoding the C region of human antibody and then is integrated into an expression vector, which is introduced into a host for antibody production (see European Patent Application EP 239400 and International Patent Publication WO 92-19759).

For the FR of human antibody ligated through CDR, those in which the complementarity determining region that forms a favorable antigen binding site are selected. When desired, amino acids in the framework region of the antibody variable region may be substituted so that the complementarity determining region of reshaped human antibody may form an appropriate antigen biding site (Sato, K. et al., Cancer Res. (1993) 53, 851-856).

For example, for chimeric antibody or humanized antibody, the C region of human antibody is used. As the C region of human antibody, there can be mentioned Cγ, and Cγ1, Cγ2, Cγ3, and Cγ4, as examples, can be used. The C region of human antibody may be modified to improve the stability of antibody or the production thereof.

Chimeric antibody consists of the variable region of antibody derived from a mammal other than the human and the C region derived from human antibody, whereas humanized antibody consists of the complementarity determining region of antibody derived from a mammal other than the human and the framework region and the C region derived from human antibody. Accordingly, antigenicity thereof in the human body has been reduced so that they are useful as antibody for use in the present invention.

As a preferred embodiment of the humanized antibody for use in the present invention, there can be mentioned humanized PM-1 antibody (see International Patent Publication WO 92-19759).

Furthermore, as a method of obtaining human antibody, a technology that employs panning with a human antibody library is known, in addition to those described above. For example, the variable region of human antibody is expressed on the surface of a phage by the phage display method as a single chain antibody (scFv) to select a phage that binds to the antigen. By analyzing the gene of the phage selected, the DNA sequence encoding the variable region of the human antibody that binds to the antigen can be determined. Once the DNA sequence of scFv that binds to the antigen is clarified, it is possible to construct an appropriate expression vector that contains said sequence and then to obtain human antibody. These methods are already known and can be found in WO 92/01047, WO 92/20791, WO 93/06213, WO 93/11236, WO 93/19172, WO 95/01438, and WO 95/15388.

Antibody genes constructed as described above may be expressed and obtained in a known method. In the case of mammalian cells, expression may be accomplished using a vector containing a commonly used useful promoter, the antibody gene to be expressed, DNA in which the poly A signal has been operably linked at 3' downstream thereof or a vector containing said DNA. Examples of the promoter/enhancer include human cytomegalovirus immediate early promoter/enhancer.

Additionally, as the promoter/enhancer which can be used for expression of antibody for use in the present invention, there can be used viral promoters/enhancers such as retrovirus, polyoma virus, adenovirus, and simian virus 40 (SV40), and promoters/enhancers derived from mammalian cells such as human elongation factor 1α (HEF1α).

For example, expression may be readily accomplished by the method of Mulligan et al. (Mulligan, R. C. et al., Nature (1979) 277, 108-114) when SV40 promoter/enhancer is used, or by the method of Mizushima et al. (Mizushima, S. and Nagata, S., Nucleic Acids Res. (1990) 18, 5322) when HEF1α promoter/enhancer is used.

In the case of E. coli, expression may be conducted by operably linking a commonly used useful promoter, a signal sequence for antibody secretion, and the antibody gene to be expressed, followed by expression thereof. As the promoter, for example, there can be mentioned lacZ promoter and araB promoter. The method of Ward et al. (Ward, E.S. et al., Nature (1989) 341, 544-546; Ward, E.S. et al., FASEB J. (1992) 6, 2422-2427) may be used when lacz promoter is used, and the method of Better et al. (Better, M. et al., Science (1988) 240, 1041-1043) may be used when araB promoter is used.

As the signal sequence for antibody secretion, when produced in the periplasm of E. coli, the pelB signal sequence (Lei, S. P. et al., J. Bacteriol. (1987) 169, 4379-4383) can be used. After separating the antibody produced in the periplasm, the structure of the antibody is appropriately refolded before use (see, for example, WO 96/30394).

As the origin of replication, there can be used those derived from SV40, polyoma virus, adenovirus, bovine papilloma virus (BPV) and the like. Furthermore, for the amplification of the gene copy number in the host cell system, expression vectors can include as selectable markers the aminoglycoside phosphotransferase (APH) gene, the thymidine kinase (TK) gene, E. coli xanthine guanine phosphoribosyl transferase (Ecogpt) gene, the dihydrofolate reductase (dhfr) gene and the like.

For the production of antibody for use in the present invention, any production system can be used.
The production system for antibody preparation comprises the in vitro or the in vivo production system. As the in vitro production system, there can be mentioned a production system which employs eukaryotic cells and the production system which employs prokaryotic cells.

When the eukaryotic cells are used, there are the production systems which employ animal cells, plant cells, or fungal cells. Known animal cells include (1) mammalian cells such as CHO cells, COS cells, myeloma cells, baby hamster kidney (BHK) cells, HeLa cells, and Vero cells, (2) amphibian cells such as Xenopus oocytes, or (3) insect cells such as sf9, sf21, and Tn5. Known plant cells include, for example, those derived from Nicotiana tabacum, which may be subjected to callus culture. Known fungal cells include yeasts such as the genus Saccharomyces, more specifically Saccharomyces cerevisiae, or filamentous fungi such as the genus Aspergillus, more specifically Aspergillus niger.

When the prokaryotic cells are used, there are the production systems which employ bacterial cells. Known bacterial cells include Escherichia coli (E. coli), and Bacillus subtilis.

By introducing via transformation the gene of the desired antibody into these cells and culturing the transformed cells in vitro, the antibody can be obtained. Culturing is conducted in the known methods. For example, as the culture liquid, DMEM, MEM, RPMI1640, and IMDM can be used, and serum supplements such as fetal calf serum (FCS) may be used in combination. In addition, antibodies may be produced in vivo by implanting cells into which the antibody gene has been introduced into the abdominal cavity of an animal and the like.

As in vivo production systems, there can be mentioned those which employ animals and those which employ plants. When animals are used, there are production systems which employ mammals and insects.

As mammals, goats, pigs, sheep, mice, and cattle can be used (Vicki Glaser, SPECTRUM Biotechnology Applications, 1993). Also as insects, silkworms can be used. When plants are used, tobacco, for example, can be used.

Antibody genes are introduced into these animals or plants, and the antibodies are produced in such animals or plants, and recovered. For example, an antibody gene is inserted into the middle of the gene encoding protein which is inherently produced in the milk such as goat β casein to prepare fusion genes. DNA fragments containing the fusion gene into which the antibody gene has been inserted are injected into a goat embryo, and the embryo is introduced into a female goat. The desired antibody is obtained from the milk produced by the transgenic goat borne to the goat who received the embryo or the offspring thereof. In order to increase the amount of milk containing the desired antibody produced by the transgenic goat, hormones may be given to the transgenic goat as appropriate. (Ebert, K.M. et al., Bio/Technology (1994) 12, 699-702).

When silkworms are used, a baculovirus into which the desired antibody gene has been inserted is infected to the silkworm, and the desired antibody can be obtained from the body fluid of the silkworm (Maeda, S. et al., Nature (1985) 315, 592-594). Moreover, when tobacco is used, the desired antibody gene is inserted into an expression vector for plants, for example pMON 530, and then the vector is introduced into a bacterium such as Agrobacterium tumefaciens. The bacterium is then infected to tobacco such as Nicotiana tabacum to obtain the desired antibody from the leaves of the tobacco (Julian, K.-C. Ma et al., Eur. J. Immunol. (1994) 24, 131-138).

When antibody is produced in vitro or in vivo production systems, as described above, DNA encoding the heavy chain (H chain) or the light chain (L chain) of antibody may be separately integrated into an expression vector and the hosts are transformed simultaneously, or DNA encoding the H chain and the L chain may be integrated into a single expression vector, and the host is transformed therewith (see International Patent Publication WO 94-11523).

Antibodies for use in the present invention may be antibody fragments or modified versions thereof as long as they are preferably used. For example, as fragments of antibody, there may be mentioned Fab, F(ab')₂, Fv or single-chain Fv (scFv) in which Fv's of H chain and L chain were ligated via a suitable linker.

Specifically, antibodies are treated with an enzyme, for example, papain or pepsin, to produce antibody fragments, or genes encoding these antibody fragments are constructed, and then introduced into an expression vector, which is expressed in a suitable host cell (see, for example, Co, M.S. et al., J. Immunol. (1994) 152, 2968-2976; Better, M. and Horwitz, A.H., Methods in Enzymology (1989) 178, 476-496; Plueckthun, A. and Skerra, A., Methods in Enzymology (1989) 178, 476-496; Lamoyi, E., Methods in Enzymology (1989) 121, 652-663; Rousseaux, J. et al., Methods in Enzymology (1989) 121, 663-66; Bird, R.E. et al., TIBTECH (1991) 9, 132-137).

scFv can be obtained by ligating the V region of H chain and the V region of L chain of antibody. In scFv, the V region of H chain and the V region of L chain are preferably ligated via a linker, preferably a peptide linker (Huston, J.S. et al., Proc. Natl. Acad. Sci. USA (1988) 85, 5879-5883). The V region of H chain and the V region of L chain in scFv may be derived from any of the above-mentioned antibodies. As the peptide linker for ligating the V regions, any single-chain peptide comprising, for example, 12-19 amino acid residues may be used.

DNA encoding scFv can be obtained using DNA encoding the H chain or the H chain V region of the above antibody and DNA encoding the L chain or the L chain V region of the above antibody as the template by amplifying the portion of the DNA encoding the desired amino acid sequence among the above sequences by the PCR technique with the primer pair specifying the both ends thereof, and by further amplifying the combination of DNA encoding the peptide linker portion and the primer pair which defines that both ends of said DNA be ligated to the H chain and the L chain, respectively.

Once DNAs encoding scFv have been constructed, an expression vector containing them and a host transformed with said expression vector can be obtained by the conventional methods, and scFv can be obtained using the resultant host by the conventional methods.

These antibody fragments can be produced by obtaining the gene thereof in a similar manner to that mentioned above and by allowing it to be expressed in a host. "Antibody" as used herein also encompasses these antibody fragments.

As modified antibodies, antibodies associated with various molecules such as polyethylene glycol (PEG) can be used. "Antibody" as used herein also encompasses these modified antibodies. These modified antibodies can be obtained by chemically modifying the antibodies thus obtained. These methods have already been established in the art.

Antibodies produced and expressed as described above can be separated from the inside or outside of the host cell and then may be purified to homogeneity. Separation and purification of the antibody for use in the present invention may be accomplished by affinity chromatography. As the column used for such affinity chromatography, there can be mentioned Protein A column and Protein G column. Examples of the carriers used in the Protein A column are Hyper D, POROS, Sepharose F. F. and the like. Alternatively, methods for separation and purification conventionally used for proteins can be used without any limitation.

Separation and purification of the antibody for use in the present invention may be accomplished by combining, as appropriate, chromatography other than the above-mentioned affinity chromatography, filtration, ultrafiltration, salting-out, dialysis and the like. Chromatography includes, for example, ion exchange chromatography, hydrophobic chromatography, gel-filtration and the like. These chromatographies can be applied into high performance liquid chromatography (HPLC). Alternatively, reverse-phase HPLC can be used.

The concentration of antibody obtained in the above can be determined by the measurement of absorbance or by ELISA and the like. Thus, when absorbance measurement is employed, a sample is appropriately diluted with PBS(-) and then the absorbance is measured at 280 nm, followed by calculation using the absorption coefficient of 1.35 OD at 1 mg/ml. When the ELISA method is used, measurement is conducted as follows. Thus, 100 µl of goat anti-human IgG (manufactured by TAG) diluted to 1 µg/ml in 0.1 M bicarbonate buffer, pH 9.6, is added to a 96-well plate (manufactured by Nunc), and is incubated overnight at 4°C to immobilize the antibody. After blocking, 100 µl each of appropriately diluted antibody of the present invention or a sample containing the antibody, or 100 µl of human IgG (manufactured by CAPPEL), as the standard, is added and incubated at room temperature for 1 hour.

After washing, 100 µl of 5000-fold diluted alkaline phosphatase-labeled anti-human IgG (manufactured by BIO SOURCE) is added, and incubated at room temperature for 1 hour. After washing, the substrate solution is added and incubated, followed by the measurement of absorbance at 405 nm using the MICROPLATE READER Model 3550 (manufactured by Bio-Rad) to calculate the concentration of the desired antibody.

The altered IL-6 for use in the present invention has an activity of binding to IL-6 receptor and does not transmit the biological activity of IL-6. Thus, the altered IL-6, though it competes with IL-6 for binding to IL-6 receptor, does not transmit the biological activity of IL-6, and thereby it blocks signal transduction by IL-6.

Altered IL-6 may be constructed through the introduction of mutation by replacing amino acid residues of the amino acid sequence of IL-6. IL-6, the source of the altered IL-6, may be of any origin, but when the antigenicity is to be considered, it is preferably human IL-6.

Specifically, the secondary structure of IL-6 is predicted using a known molecular modeling program of the amino acid sequence, for example WHATIF (Vriend et al., J. Mol. Graphics (1990), 8, 52-56), and the overall effects on the amino acid residue to be replaced is evaluated. After an appropriate amino acid residue has been determined, mutation is introduced to effect amino acid substitution by the commonly used polymerase chain reaction (PCR) method using a vector containing the base sequence encoding human IL-6 gene as a template thereby to obtain a gene encoding an altered IL-6. This is then integrated, as desired, into an appropriate expression vector, from which the altered IL-6 can be obtained according to the expression, production and purification methods of said recombinant antibody.

Specific examples of the altered IL-6 are disclosed in Brakenhoff et al., J. Biol. Chem. (1994) 269, 86-93, and Savino et al., EMBO J. (1994) 13, 1357-1367, WO 96-18648, and WO 96-17869.

The IL-6 partial peptide or the IL-6 receptor partial peptide for use in the present invention has an activity of binding to IL-6 receptor or IL-6, respectively, and does not transmit the biological activity of IL-6. Thus, the IL-6 partial peptide or the IL-6 receptor partial peptide specifically inhibits the binding of IL-6 to IL-6 receptor by binding to IL-6 receptor or IL-6, respectively, and thereby capturing it. As a result, they do not transmit the biological activity of IL-6, and thus block signal transduction of IL-6.

The IL-6 partial peptide or the IL-6 receptor partial peptide is a peptide comprising some or all of the amino acid sequence of the region involved in the binding to IL-6 and IL-6 receptor in the amino acid sequence of IL-6 or IL-6 receptor. Such a peptide generally comprises 10 - 80, preferably 20 - 50, more preferably 20 - 40 amino acid residues.

The IL-6 partial peptide or the IL-6 receptor partial peptide can be constructed by specifying the region involved in the binding to IL-6 and IL-6 receptor in the amino acid sequence of IL-6 or IL-6 receptor, and by producing some or all of the amino acid sequence by a conventional method such as a genetic engineering technology or a peptide synthesis method.

In order to prepare the IL-6 partial peptide or the IL-6 receptor partial peptide by a genetic engineering technology, the DNA sequence encoding the desired peptide is integrated into an expression vector, from which the peptide can be obtained by the expression, production, and purification methods of said recombinant antibody.

Preparation of the IL-6 partial peptide or the IL-6 receptor partial peptide by the peptide synthesis method can be effected using a method commonly used in peptide synthesis such as solid phase synthesis or liquid phase synthesis.

Specifically, the method described in Zoku-Iyakuhin no Kaihatsu (Sequel to Development of Pharmaceuticals), Vol. 14, Peputido Gousei (Peptide Synthesis), edited by Haruaki Yajima, Hirokawa Shoten, 1991, may be used. The solid phase synthesis method used includes, for example, a reaction in which an amino acid corresponding to the C-terminal of the peptide to be synthesized is coupled to a support which is insoluble in organic solvents, and then an amino acid in which α-amino group or a side chain functional group has been protected with an appropriate protecting group is condensed, one amino acid at a time, from the C-terminal to the N-terminal direction, and a reaction in which said protecting group of the α-amino group of the amino acid or the peptide coupled to the resin is eliminated are alternately repeated to elongate the peptide chain. The solid phase peptide synthesis methods are divided into the Boc method and the Fmoc method depending on the type of protecting group to be used.

After the synthesis of the desired peptide is complete, a deprotection reaction and a reaction for cleaving the peptide chain from the support are carried out. For cleavage from the peptide chain, hydrogen fluoride or trifuluoromethane sulfonic acid in the Boc method, and TFA in the Fmoc method are generally used.
In the Boc method, for example, the above protected peptide resin is treated in hydrogen fluoride in the presence of anisole. Subsequently, the protecting group is eliminated and the peptide is recovered by cleaving from the support. By lyophilizing, a crude peptide can be obtained. On the other hand, in the Fmoc method, the deprotection reaction and the cleavage reaction of the peptide from the support may be performed in TFA for example, in a procedure similar to the above.

The crude peptide thus obtained can be applied to HPLC for its separation and purification. Its elution can be carried out in a water-acetonitrile solvent system that is commonly used for protein purification under an optimum condition. The fraction corresponding to the peak of the profile of the chromatography obtained is collected and lyophilized. The peptide fraction thus purified is identified by subjecting it to the analysis of molecular weight by mass spectroscopic analysis, the analysis of amino acid composition, or the analysis of amino acid sequence, and the like.

Specific examples of the IL-6 partial peptide or the IL-6 receptor partial peptide are disclosed in Japanese Unexamined Patent Publication (Kokai) 2-188600, Japanese Unexamined Patent Publication (Kokai) 7-324097, Japanese Unexamined Patent Publication (Kokai) 8-311098, and United States Patent Publication US 5210075.

The activity of the IL-6 antagonist for use in the present invention of blocking signal transduction of IL-6 can be evaluated using a conventionally known method. Specifically, the IL-6-dependent human myeloma cell line (S6B45, KPMM2), human Lennert's T-lymphoma cell line KT3, or IL-6-dependent cell MH60.BSF2 is cultured, to which IL-6 is added, and the activity can be evaluated using the incorporation of ³H-thymidine into the IL-6-dependent cell with the coexistence of the IL-6 antagonist.

Alternatively, U266, an IL-6 receptor-expressing cell, may be cultured, to which ¹²⁵I-labeled IL-6 is added and an IL-6 antagonist is added at the same time, and then the ¹²⁵I-labeled IL-6 bound to the IL-6 receptor-expressing cell is determined. In the above assay system, a negative control group containing no IL-6 antagonists, in addition to the group in which an IL-6 receptor antagonist is present, is set up, and the results obtained for them are compared to evaluate the IL-6-inhibiting activity of the IL-6 antagonist.

As described in the Example below, anti-IL-6 receptor antibody exhibited a therapeutic effect in patients with spinal cord injury. The subject to be treated in the present invention is a mammal. The subject mammal to be treated is preferably a human.

The therapeutic agents for spinal cord injury of the present invention may be administered, either orally or parenterally, systemically or locally. For example, intravenous injection such as drip infusion, intramuscular injection, intraperitoneal injection, subcutaneous injection, suppositories, intestinal lavage, oral enteric coated tablets, and the like can be selected, and the method of administration may be chosen, as appropriate, depending on the age and the conditions of the patient.

The effective dosage is chosen from the range of 0.01 mg to 100 mg per kg of body weight per administration. Alternatively, the dosage in the range of 1 to 1000 mg, preferably 5 to 50 mg per patient may be chosen. Preferred dosages and preferred methods of administration are such that, in the case of anti-IL-6 receptor antibody, the amounts wherein free antibody is present in the blood are effective dosages. In specific examples, 0.5 mg to 40 mg per kg of body weight, preferably 1 mg to 20 mg, per month (4 weeks) are administered in one to several doses, for example in the administration schedule of twice per week, once per week, once every two weeks, once every four weeks and the like by intravenous injection such as drip infusion and subcutaneous injection. The administration schedule can be adjusted by observing the disease conditions and blood levels of laboratory tests by, for example, extending the administration interval from twice per week or once per week to once per two weeks, once per three weeks, once per four weeks, and the like.

The therapeutic agents for spinal cord injury of the present invention may contain pharmaceutically acceptable carriers or additives depending on the route of administration. Examples of such carriers or additives include water, a pharmaceutical acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, gelatin, agar, diglycerin, propylene glycol, polyethylene glycol, Vaseline, paraffin, stearyl alcohol, stearic acid, human serum albumin (HSA), mannitol, sorbitol, lactose, a pharmaceutically acceptable surfactant and the like. Additives used are chosen from, but not limited to, the above or combinations thereof depending on the dosage form.

### Examples

The present invention will now be explained in more details with reference to the examples and reference examples. It should be noted, however, that the present invention is not limited to them in any way.

### Example 1

### Materials and Methods

### Animals:

Adult (18-22g) female c57BL/6J mice were used in all experiment groups.

### Spinal cord injury:

Female mice were anesthetized by the intraperitoneal injection of ketamine (100 mg/kg) and xylazine (10 mg/kg). The back was shaved, a 20 mm midline skin incision was made, and then the spine was exposed. After the thoracic region of the spine was exposed by separation to the sides of the back muscle, the spinous process of the T7-T13 vertebra was exposed. Laminectomy was made at the ninth thoracic spine level to expose the spinal cord taking care not to injure the dura mater.
The spine was stabilized with forceps and clamps on the T7 and T11 spinous processes and the ligament. After the animal body was floated by lowering the stage, spinal cord injury (SCI) was made by the NYU impactor. A 3g weight (the apex with a diameter of 1.2 mm) was dropped from a height of 25 mm to the T9 level spinal cord. Muscles and the incised part were closed in layers, and the animals were placed in a temperature-controlled chamber until temperature control is reestablished. Urination by manual bladder expulsion was performed twice per day until spontaneous urination was established.

### Protocol and the injection of rat anti-mouse IL-6 receptor mAb (MR16-1) :

Immediately after injury, 100 µg of MR16-1 per gram of mouse body weight was administered by a single intraperitoneal injection (the MR16-1 group, n=15), and the control group received the same amount of rat IgG by intraperitoneal injection (the control group, n=15). For histological and immunohistochemical analyses of the both groups, the intraperitoneal injection of BrdU (50 mg/kg body weight) was performed for two weeks from the day of surgery in order to label the dividing cells.

### Evaluation of motor function:

Using three different tests, the present inventors evaluated the recovery of motor function after injury. The functional evaluation was continued to week 6 after injury.

Evaluation of motor function of the lower limbs: In order to evaluate the functional effect of SCI, the present inventors performed a motor function evaluation by the Basso-Beattie-Bresnahan (BBB) score which has been commonly and widely used. Three different testers evaluated individual animals for four minutes in a double blind manner, and scored (0-21) the function of individual lower limbs as defined. All tests were recorded in videotapes.

SCANET: SCANET is an automated analytical system of animal movement comprising a cage equipped with an infrared sensor frame. This monitors minor (M1) and major (M2) lateral and vertical motions (RG), i.e. the number of times of standing up, which quantitates the amount of motion spontaneously performed by the animal in a given time. In particular, it is said that there is a positive statistical relationship between the RG score and the BBB score.

Rotarod treadmill: The coordinated motion of four limbs was evaluated by placing a mouse on a revolving rod device comprising a plastic rod so as to force the mice to walk. The mouse was placed on the revolving rod at speeds of 5, 10 and 15 rpm, and the latent time until it dropped was monitored for 120 seconds. The function of coordinated motion was each evaluated from the mean value and the maximum.

### Immunohistochemistry:

For histological examination, mice were anesthetized by inhalation of diethyl ether, and 4% paraformaldehyde was transcardiacly perfused, and the mice were fixed.
The spinal cord was extracted, and postfixed with 4% paraformaldehyde at room temperature for a few hours.
The tissue sample was immersed in 10% sucrose at 4°C for 24 hours, and placed in 30% sucrose for 48 hours prior to embedding it in the OTC compound. The embedded tissue was frozen in liquid nitrogen and stored at -80°C. The cryosections were made by sagittal section and axial section at a thickness of 20 micrometers, and stained with the HE stain or the immunofluoro double stain.

For the immunofluoro double stain experiment, the spinal cord section was blocked in 0.03% Triton X-100 and 10% normal goat serum in 0.01M PBS (pH 7.4) for 30 minutes. As the primary antibody, rabbit anti-GFAP antibody, rat anti-Brd-U antibody and human anti-Hu antibody (as a neuron marker) were used, and incubated overnight at 4°C. As the secondary antibody FITC-conjugated rabbit IgG antibody and Texas Red-conjugated rat antibody were used, and double-stained. The slides were washed, wet-fixed, and analyzed under a fluoromicroscope.

### Western blot analysis:

Twelve hours after injury creation (n=4 for each group), a 8 mm segment of the spinal cord (from the center of injury, 4 mm proboscis side and 4 mm caudal side) was excised, which was homogenized in a MAPK bacteriolytic buffer containing a protease inhibitor, and sonicated followed by centrifugation at 15,000 rpm. Protein was separated from the supernatant of each sample by SDS-PAGE, and blotted to a poly(difluoride) vinylidene membrane by electrophoresis. After the membrane was blocked in a TBST buffer containing 5% defatted milk, 150 mM NaCl and 0.05% Tween 20 (pH 7.5) at room temperature for one hour, either of polyclonal rabbit anti-stat3 antibody or rabbit anti-phosphorylated stat3 antibody or rabbit anti-IL-6Rα antibody was used as the primary antibody, and then incubated together with HRP-conjugated anti-rabbit IgG antibody as the secondary antibody. After developing into a film by an automated developer, it was quantitated by the α-imager.

### Result:

### (1) Evaluation of motor function of lower limbs

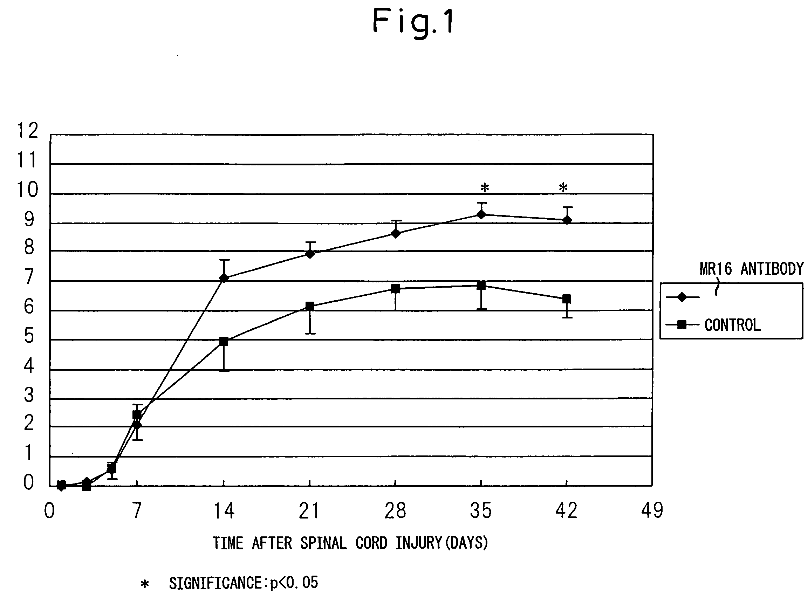

For 15 spinal cord injured mice that received anti-IL-6 receptor antibody (MR16) and 15 spinal cord injured mice (control) that did not receive the above antibody, mean values of BBB scores are shown in a graph in Fig. 1. After spinal cord injury, on day 7 and thereafter, the recovery of motion was good in the group of mice that received MR16 antibody, and on week 5 and week 6 a significant difference was noted.

### (2) SCANET

For 15 spinal cord injured mice that received anti-IL-6 receptor antibody (MR16) and 15 spinal cord injured mice (control) that did not receive the above antibody, lateral motion and the number of times of standing up were compared, with a result that no significant difference was noted in lateral motion, but the standing up motion was observed in 12 of 15 spinal cord injured mice that received anti-IL-6 receptor antibody (MR16) whereas it was only observed in 3 of 15 spinal cord injured mice (control) that did not receive the above antibody. This difference was significant by Fisher's exact probability test with p<0.05.

### (3) Rotarod treadmill

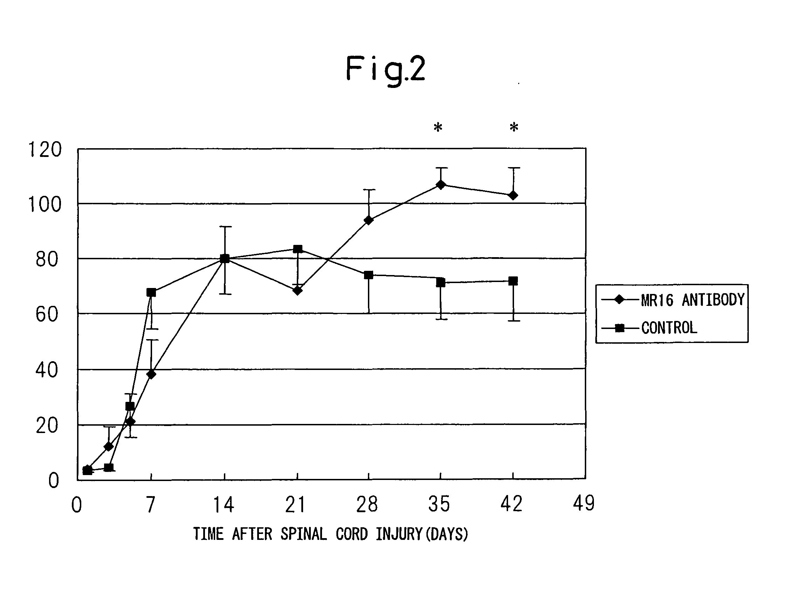

For 15 spinal cord injured mice that received anti-IL-6 receptor antibody (MR16) and 15 spinal cord injured mice (control) that did not receive the above antibody, the above experiment was performed, with a result that no significant difference was noted when the revolving speed of the rod was 10 rpm (10 revolutions per minute) and 15 rpm, whereas at 5 rpm, as shown in Fig. 2, on day 358 and thereafter after spinal cord injury, the recovery of the spinal cord injured mice that received anti-IL-6 receptor antibody (MR16) was significantly (p<0.05) higher than that of the spinal cord injured mice (control) that did not receive the above antibody.

### (4) Immunohistochemical examination

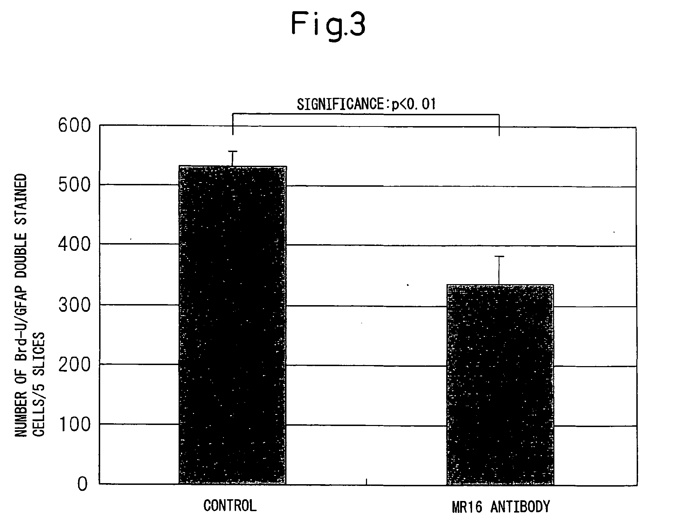

Despite the presence of inherent neural stem cells in the adult spinal cord, the repair of the spinal cord due to the differentiation of the cells does not occur. This is probably because the inherent neural stem cells differentiate into glia precursor cells and further into astrocytes in the injured spinal cord and thus do not differentiate into neuronal cells. When the formation of reactive astrocytes was counted by an immunofluorescent method using anti-GFAP antibody and anti-BrDU antibody for the spinal cord of the injured part of four spinal cord injured mice that received anti-IL-6 receptor antibody (MR16) and the injured part of four spinal cord injured mice (control) that did not receive the above antibody, as shown in Fig. 3, the administration of the above antibody significantly reduced the formation of reactive astrocytes (p<0.01).

### (5) Western blot analysis

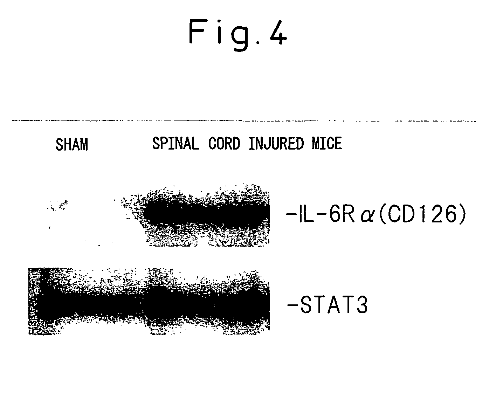

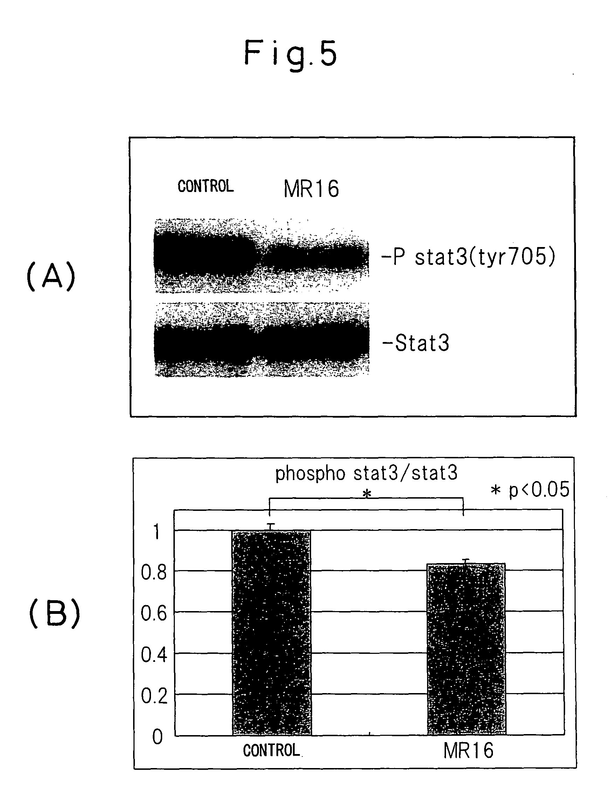

For four spinal cord injured mice and four spinal cord injured mice (control), the expression of IL-6 receptor at 12 hours after spinal cord injury was investigated by Western blot analysis. The result is shown in Fig. 4. The expression of IL-6 receptor was only observed in the spinal cord injured mice. Also, as shown in Fig. 5, the administration of MR16 suppressed the amount of phosphorylated STAT3, indicating that the intraperitoneally administered MR16 acted in the spinal cord.

The foregoing confirmed that IL-6 antagonists promote the repair of spinal cord injury.

### Reference example 1. Preparation of human soluble IL-6 receptor

Soluble IL-6 receptor was prepared by the PCR method using a plasmid pBSF2R.236 containing cDNA that encodes IL-6 receptor obtained according to the method of Yamasaki et al., (Yamasaki, K. et al., Science (1988) 241, 825-828). Plasmid pBSF2R.236 was digested with a restriction enzyme Sph I to obtain the cDNA of IL-6 receptor, which was then inserted into mp18 (manufactured by Amersham). Using a synthetic oligoprimer designed to introduce a stop codon into the cDNA of IL-6 receptor, a mutation was introduced into the cDNA of IL-6 receptor by the PCR method using the in vitro Mutagenesis System (manufactured by Amersham). The procedure resulted in the introduction of a stop codon to the amino acid at position 345, and gave cDNA encoding soluble IL-6 receptor.

In order to express the cDNA of soluble IL-6 receptor in CHO cells, it was ligated to a plasmid pSV (manufactured by Pharmacia) to obtain a plasmid pSVL344. The cDNA of soluble IL-6 receptor that was cleaved with Hind III-Sal I was inserted to plasmid pECEdhfr containing the cDNA of dhfr to obtain a plasmid pECEdhfr344 that can be expressed in the CHO cells.

Ten µg of plasmid pECEdhfr344 was transfected to a dhfr-CHO cell line DXB-11 (Urlaub G. et al., Proc. Natl. Acad. Sci. USA (1980) 77, 4216-4220) by the calcium phosphate precipitation method (Chen C. et al., Mol. Cell. Biol. (1987) 7, 2745-2751). The transfected CHO cells were cultured for 3 weeks in a nucleoside-free α MEM selection medium containing 1 mM glutamine, 10% dialyzed FCS, 100 U/ml penicillin, and 100 µg/ml streptomycin.

The selected CHO cells were screened by the limiting dilution method to obtain a single CHO cell clone. The CHO cell clone was amplified in 20 nM - 200 nM methotrexate (MTX) to obtain a CHO cell line 5E27 that produces human soluble IL-6 receptor. The CHO cell line 5E27 was cultured in an Iscov-modified Dulbecco's medium (IMDM, manufactured by Gibco) containing 5% FBS. The culture supernatant was collected and the concentration of soluble IL-6 receptor in the culture supernatant was determined by ELISA. The result confirmed that soluble IL-6 receptor is present in the culture supernatant.

### Reference example 2. Preparation of anti-human IL-6 antibody

Ten µg of the recombinant IL-6 (Hirano et al., Immunol. Lett., (1988) 17, 41) was used for immunizing BALB/c mice together with Freund's complete adjuvant, and this was repeated every week until anti-IL-6 antibody could be detected in the serum. Immune cells were extracted from local lymph nodes and were then fused with a myeloma cell line P3U1 using polyethylene glycol 1500. Hybridomas were selected according to the method of Oi et al. (Selective Methods in Cellular Immunology, W.H. Freeman and Co., San Francisco, 351, 1980) that employs the HAT medium, and the hybridoma that produces anti-human IL-6 antibody was established.

The hybridoma that produces anti-human IL-6 antibody was subjected to the IL-6 binding assay as follows.
Thus, a 96-well microtiter plate made of flexible polyvinyl (manufactured by Dynatech Laboratories, Inc., Alexandria, VA) was coated with 100 µl of goat anti-mouse Ig (10 µl/ml, manufactured by Cooper Biomedical, Inc., Malvern, PA) overnight at 4°C in 0.1 M carbonate-hydrogen carbonate buffer, pH 9.6. Subsequently, the plate was treated with 100 µl of PBS containing 1% bovine serum albumin (BSA) at room temperature for 2 hours.

After washing it in PBS, 100 µl of the hybridoma culture supernatant was added to each well, and then was incubated overnight at 4°C. The plate was washed, ¹²⁵I-labeled recombinant IL-6 was added to each well to a concentration of 2000 cpm/0.5 ng/well, and then the radioactivity of each well after washing was determined by a gamma counter (Beckman Gamma 9000, Beckman Instruments, Fullerton, CA). Of 216 hybridoma clones, 32 were positive in the IL-6 binding assay. From these clones, stable MH166.BSF2 was finally obtained. Anti-IL-6 antibody MH166 produced by said hybridoma has a subtype of IgG1 κ.

Then, the IL-6-dependent mouse hybridoma clone MH60.BSF2 was used to examine a neutralizing activity with respect to the growth of the hybridoma by MH166 antibody. MH60.BSF2 cells were dispensed to 1 × 10⁴/200 µl/well, and samples containing MH166 antibody were added thereto, cultured for 48 hours, 0.5 µCi/well of ³H-thymidine (New England Nuclear, Boston, MA) was added, and the culturing was continued for further 6 hours. The cells were placed on a glass filter paper and were treated by the automatic harvester (Labo Mash Science Co., Tokyo, Japan). As the control, rabbit anti-IL-6 antibody was used.

As a result, MH166 antibody inhibited, in a dose dependent manner, the incorporation of ³H-thymidine of MH60.BSF2 cells induced by IL-6. This revealed that MH166 antibody neutralizes the activity of IL-6.

### Reference example 3. Preparation of anti-human IL-6 receptor antibody

Anti-IL-6 receptor antibody MT18 prepared by the method of Hirata et al. (Hirata, Y. et al. J. Immunol., (1989) 143, 2900-2906) was bound to CNBr-activated Sepharose 4B (manufactured by Pharmacia Fine Chemicals, Piscataway, NJ) according to the attached regimen, and IL-6 receptor (Yamasaki, K. et al., Science (1988) 241, 825-828) was purified. A human myeloma cell line U266 was solubilized with 1 mM p-para-aminophenyl methane sulfonyl fluoride hydrochloride (manufactured by Wako Chemicals) (digitonin buffer) containing 1% digitonin (manufactured by Wako Chemicals), 10 mM triethanolamine (pH 7.8) and 0.15 M NaCl, and mixed with MT18 antibody bound to Sepharose 4B beads. Then, the beads were washed six times with the digitonin buffer to prepare the partially purified IL-6 receptor to be used for immunization.

BALB/c mice were immunized four times every ten days with the above partially purified IL-6 receptor obtained from 3 × 10⁹ U266 cells, and then a hybridoma was prepared using a standard method. The hybridoma culture supernatant from the growth-positive well was tested for its activity of binding to IL-6 receptor according to the method described below. 5 × 10⁷ U266 cells were labeled with ³⁵S-methionine (2.5 mCi) and were solubilized with the above digitonin buffer. The solubilized U266 cells were mixed with a 0.04 ml volume of MT18 antibody bound to Sepharose 4B beads, and then were washed six times with the digitonin buffer. ³⁵S-methionine-labeled IL-6 receptor was eluted with 0.25 ml of the digitonin buffer (pH 3.4) and was neutralized in 0.025 ml of 1M Tris (pH 7.4) .

0.05 ml of the hybridoma culture supernatant was mixed with 0.01ml of Protein G Sepharose (manufactured by Pharmacia). After washing, Sepharose was incubated with 0.005 ml ³⁵S-labeled IL-6 receptor solution prepared as described above. The immunoprecipitate was analyzed by SDS-PAGE to investigate the hybridoma culture supernatant that reacts with IL-6 receptor. As a result, a reaction-positive hybridoma clone PM-1 (FERM BP-2998) was established. The antibody produced from the hybridoma PM-1 has a subtype of IgG1 κ.

The inhibitory activity of the antibody produced by the hybridoma PM-1 on the binding of IL-6 to human IL-6 receptor was studied using the human myeloma cell line U266. A human recombinant IL-6 was prepared from E. coli (Hirano et al., Immunol. Lett., (1988) 17, 41-45), and was labeled with ¹²⁵I using the Bolton-Hunter reagent (New England Nuclear, Boston, MA) (Taga, T. et al., J. Exp. Med. (1987) 166, 967-981).

4 × 10⁵ U266 cells were cultured with the 70% (v/v) culture supernatant of hybridoma PM-1 together with 14,000 cpm of ¹²⁵I-labeled IL-6 for one hour. Seventy µl of the sample was layered on 300 µl FCS in a 400 µl microfuge polyethylene tube. After centrifugation, the radioactivity of the cell was determined.

The result revealed that the antibody produced by the hybridoma PM-1 inhibits the binding of IL-6 to IL-6 receptor.

### Reference example 4. Preparation of mouse anti-IL-6 receptor antibody

A monoclonal antibody directed against mouse IL-6 receptor was prepared according to the method described in Saito, et al., J. Immunol. (1991) 147, 168-173.

The CHO cells that produce mouse soluble IL-6 receptor were cultured in the IMDM culture liquid containing 10% FCS. From the culture supernatant, mouse soluble IL-6 receptor was purified using an affinity column in which anti-mouse IL-6 receptor antibody RS12 (see Saito, et al., supra) had been fixed to Affigel 10 gel (manufactured by Biorad).

The mouse soluble IL-6 receptor (50 µg) thus obtained was mixed with Freund's complete adjuvant, which was then injected to the abdomen of Wistar rats. From two weeks after the administration, the animals were boosted with Freund's incomplete adjuvant. On day 45, rat spleen cells were harvested, and about 2 × 10⁸ cells thereof were fused with 1 × 10⁷ mouse myeloma cells P3U1 using a 50% PEG1500 (manufactured by Boehringer Mannheim) according to the conventional method, and then were screened by the HAT culture medium.

After the hybridoma culture supernatant was added to the plate coated with rabbit anti-rat IgG antibody (manufactured by Cappel), mouse soluble IL-6 receptor was reacted. Subsequently, using rabbit anti-mouse IL-6 receptor antibody and alkaline phosphatase-labeled sheep anti-rabbit IgG, hybridomas producing antibody directed against mouse soluble IL-6 receptor were screened by ELISA. Hybridoma clones for which antibody production was confirmed were subscreened twice to obtain a single hybridoma clone. The clone was designated as MR16-1.

The neutralizing activity of the antibody produced by the hybridoma on signal transduction of mouse IL-6 was examined by ³H-thymidine incorporation using MH60.BSF2 cells (Matsuda, T. et al., J. Immunol. (1988) 18, 951-956). To a 96-well plate, MH60.BSF2 cells were prepared at 1 × 10⁴ cells/200 µl/well. To the plate were added 10 pg/ml mouse IL-6 and MR16-1 antibody or RS12 antibody at 12.3 - 1000 ng/ml, then they were cultured at 37°C and 5% CO₂ for 44 hours, and then 1 µCi/well of ³H-thymidine was added. After 4 hours, the incorporation of ³H-thymidine was measured. As a result, it was found that MR16-1 antibody suppressed the incorporation of ³H-thymidine by the MH60.BSF2 cells.

Thus, it was demonstrated that the antibody produced by the hybridoma MR16-1 (FERM BP-5875) inhibits the binding of IL-6 to IL-6 receptor.

## Claims

1. A therapeutic agent for spinal cord injury comprising an interleukin-6 (IL-6) antagonist as an active ingredient.

2. The therapeutic agent for spinal cord injury according to claim 1 wherein said IL-6 antagonist is an antibody against IL-6 receptor.

3. The therapeutic agent for spinal cord injury according to claim 2 wherein said antibody is a monoclonal antibody.

4. The therapeutic agent for spinal cord injury according to claim 2 or 3 wherein said antibody is a monoclonal antibody against human IL-6 receptor.

5. The therapeutic agent for spinal cord injury according to claim 2 or 3 wherein said antibody is a monoclonal antibody against mouse IL-6 receptor.

6. The therapeutic agent for spinal cord injury according to any one of claims 1 to 4 wherein said antibody is a recombinant antibody.

7. The therapeutic agent for spinal cord injury according to any one of claims 1 to 6 wherein said monoclonal antibody against human IL-6 receptor is PM-1 antibody.

8. The therapeutic agent for spinal cord injury according to claim 5 wherein said monoclonal antibody against mouse IL-6 receptor is MR16-1 antibody.

9. The therapeutic agent for spinal cord injury according to any one of claims 1 to 8 wherein said antibody is a chimeric antibody, a humanized antibody, or a human antibody against IL-6 receptor.

10. The therapeutic agent for spinal cord injury according to claim 9 wherein said humanized antibody is a humanized PM-1 antibody.

11. A modulator of differentiation of neural stem cells comprising an interleukin-6 (IL-6) antagonist as an active ingredient.

12. An inhibitor of differentiation into glia cells comprising an interleukin-6 (IL-6) antagonist as an active ingredient.

13. The use of an interleukin-6 (IL-6) antagonist for the manufacture of a therapeutic agent for spinal cord injury.

14. The use according to claim 13 wherein said IL-6 antagonist is an antibody against IL-6 receptor.

15. The use according to claim 14 wherein said antibody is a monoclonal antibody.

16. The use according to claim 13 or 14 wherein said antibody is a monoclonal antibody against human IL-6 receptor.

17. The use according to claim 14 or 15 wherein said antibody is a monoclonal antibody against mouse IL-6 receptor.

18. The use according to any one of claims 13 to 16 wherein said antibody is a recombinant antibody.

19. The use according to any one of claims 13 to 18 wherein said monoclonal antibody against human IL-6 receptor is PM-1 antibody.

20. The use according to claim 17 wherein said monoclonal antibody against mouse IL-6 receptor is MR16-1 antibody.

21. The use according to any one of claims 13 to 20 wherein said antibody is a chimeric antibody, a humanized antibody, or a human antibody against IL-6 receptor.

22. The use according to claim 21 wherein said humanized antibody is a humanized PM-1 antibody.

23. The use of interleukin-6 (IL-6) antagonist for the manufacture of a modulator of differentiation of neural stem cells.

24. The use of interleukin-6 (IL-6) antagonist for the manufacture of an inhibitor of differentiation into glia cells.

25. A therapeutic method for spinal cord injury of a subject which comprises administering an interleukin-6 (IL-6) antagonist to the subject.

26. The method according to claim 25 wherein said IL-6 antagonist is an antibody against IL-6 receptor.

27. The method according to claim 26 wherein said antibody is a monoclonal antibody.

28. The method according to claim 26 or 27 wherein said antibody is a monoclonal antibody against human IL-6 receptor.

29. The method according to claim 26 or 27 wherein said antibody is a monoclonal antibody against mouse IL-6 receptor.

30. The method according to any one of claims 25 to 28 wherein said antibody is a recombinant antibody.

31. The method according to any one of claims 25 to 30 wherein said monoclonal antibody against human IL-6 receptor is PM-1 antibody.

32. The method according to claim 29 wherein said monoclonal antibody against mouse IL-6 receptor is MR16-1 antibody.

33. The method according to any one of claims 25 to 32 wherein said antibody is a chimeric antibody, a humanized antibody, or a human antibody against IL-6 receptor.

34. The method according to claim 33 wherein said humanized antibody is a humanized PM-1 antibody.

35. A method of modulating the differentiation of neural stem cells of a subject which comprises administering an interleukin-6 (IL-6) antagonist to the subject.

36. A method of inhibiting differentiation into glia cells, in a subject, which comprises administering an interleukin-6 (IL-6) antagonist to the subject.
